# DEMANDE DE BREVET EUROPEEN

(11) **EP 1 234 590 A1**
(43) Date de publication de la demande: **28.08.2002**
(21) Numéro de dépôt: 02352003.4
(22) Date de dépôt: 22.02.2002
(51) Int. Cl.: A61M 1/16, B01D 61/02

(54) **Installation de dialyse multipostes**

(30) Priorité: 23.02.2001 FR 0102486
(71) Demandeur: Lopez, Société Anonyme, 31200 Toulouse (FR)
(72) Inventeur: Lopez, Fernand, 31200 Toulouse (FR)
(74) Mandataire: Cabinet BARRE LAFORGUE & associés

(57) **Abrégé**

L'invention concerne une installation de dialyse multipostes comprenant une pompe (12) disposée sur une conduite principale (40, 11) et adaptée pour élever la pression de l'eau à une pression Pₒₛₘ apte à permettre un traitement par osmose inverse, et pour chaque générateur de dialyse (18), une branche de distribution (15, 28, 33), raccordée à la conduite principale (40, 11), sur laquelle sont disposés une unité d'osmose inverse (17) renfermant au moins une membrane semi-perméable d'osmose inverse (19, 25), et un connecteur hydraulique (31) et un clapet (35) taré à une pression de tarage prédéterminée Pₐ inférieure à la pression Pₒₛₘ, définissant la pression d'alimentation du générateur de dialyse (18), ledit connecteur et ledit clapet étant adaptés pour dériver, de la branche de distribution (15, 28, 33), un débit d'eau d'alimentation à la pression de tarage Pₐ vers le générateur de dialyse (18).

## Description

L'invention concerne une installation de dialyse multipostes pour centre ou antenne d'auto-dialyse.

Les installations de dialyse multipostes sont destinées à l'épuration du sang de malades et comprennent plusieurs générateurs de dialyse, chacun affecté à un malade, reliés par une conduite ou boucle d'alimentation à des moyens de purification d'eau situés dans un local technique isolé attenant à la salle de traitement, et composés généralement de filtres, d'un ou d'adoucisseurs, et d'un osmoseur.

De telles installations de dialyse sont soumises à des normes de sécurité draconiennes visant à garantir contre tout accident susceptible de nuire à la santé des patients traités par contamination du sang de ces derniers. A cet effet, à titre essentiel, ces installations doivent être notamment conçues de sorte que l'eau délivrée au niveau des générateurs présente une grande pureté.

Les normes en terme de sécurité s'avèrent, en outre, être de plus en plus sévères notamment dans les installations modernes qui sont équipées de membranes de dialyse performantes bio-compatibles, c'est-à-dire très poreuses, exigeant une eau de très haute qualité ; des impuretés même légères apparaissant dans le dialysat peuvent traverser la membrane et contaminer le sang du malade.

Pour remplir ces exigences, le matériel utilisé dans les installations de dialyse devient de plus en plus complexe, dans l'espoir d'être de plus en plus performant, et nécessite de lourds investissements. De même, par voie de conséquence, les frais de maintenance représentent un poste important en terme de coût de revient, du fait d'une part du coût des pièces détachées qui sont souvent chères, et, d'autre part, du temps de main d'oeuvre requis pour les dépannages qui nécessitent l'intervention de techniciens qualifiés.

A titre d'exemple, concernant le matériel utilisé, les osmoseurs actuels qui représentent l'élément le plus complexe et le plus délicat de la chaîne car destiné, à partir d'une eau filtrée et adoucie, à fournir toute l'eau pure d'alimentation de l'ensemble des générateurs, sont accompagnés d'un équipement technique (hydraulique, électrique, électronique et informatique) très sophistiqué en vue d'assurer et surveiller leur fonctionnement. A noter notamment que les dernières directives imposent que soit prévue une télésurveillance permettant aux infirmières, depuis la salle de soins, d'être averties de toute anomalie pouvant se produire sur l'osmoseur.

Toujours concernant le matériel, un soin tout particulier est également apporté aux boucles d'alimentation, tant concernant la qualité des matériaux qui les composent que leur conception, afin que l'eau pure qui circule dans ces dernières, aussi fragile que précieuse, ne soit pas altérée avant d'atteindre les générateurs.

Par ailleurs, l'ensemble de l'installation doit obligatoirement être régulièrement désinfecté, la boucle d'alimentation étant particulièrement concernée et surveillée, et faisant l'objet de traitements de désinfection de plus en plus sophistiqués et donc de plus en plus coûteux. Ainsi, notamment, se sont développées, depuis quelques années, des techniques de désinfection coûteuses à la chaleur, voire même à la vapeur d'eau, qui imposent en outre de réaliser les boucles d'alimentation en acier inoxydable de haute qualité.

Il ressort donc de ce qui précède, qu'à des fins louables d'éviter tout risque d'accident, les installations de dialyse actuelles deviennent de plus en plus coûteuses, tant en terme d'investissement en matériel qu'en terme de coût de fonctionnement.

Et, malgré cette surenchère de technicité et de précautions, il s'avère que les risques d'accident subsistent. Or, du fait de la conception de ces installations, lorsqu'un tel accident survient, tous les générateurs se trouvent pollués en même temps et donc tous les patients concernés, avec des conséquences de divers degrés d'importance et de gravité, telles que :
- perturbation de la santé de tous les patients,
- obligation d'intervenir immédiatement pour remédier au problème, soit technique s'il s'agit d'un dysfonctionnement de l'osmoseur, soit biochimique si des germes se sont développés dans la boucle d'alimentation,
- obligation dans la majorité des cas de transférer les patients vers un autre centre de dialyse.

A l'heure actuelle, la seule alternative proposée pour réduire les risques d'accident, et les conséquences qui découlent de ces derniers, a consisté à envisager d'avoir recours à un osmoseur complet disposé à proximité de chaque générateur et alimentant individuellement ce dernier. Toutefois, la mise en oeuvre d'une telle solution dans une salle de traitement multipostes s'est avérée présenter plusieurs inconvénients qui ont conduit à son échec :
- encombrement important,
- équipement électrique secteur pour chaque osmoseur,
- échauffement de l'atmosphère, surtout en été, par l'ensemble des moteurs et pompes fonctionnant simultanément,
- bruit cumulé occasionné par les moteurs et pompes fonctionnant simultanément,
- interférences et possibilités de perturbations électromagnétiques,
- coût élevé de l'investissement,
- renouvellement périodique de pièces d'usure, notamment de pompes,
- pannes diverses inévitables et frais de maintenance en découlant.

Par conséquent, les seules évolutions qu'ont connu les installations de dialyse multipostes depuis leur création ont abouti à concevoir de véritables usines miniatures de plus en plus complexes, de plus en plus chères, et nécessitant un entretien et une maintenance de plus en plus rigoureux et contraignants.

A l'encontre de cette tendance constante, la présente invention a pour principal objectif de fournir une installation de dialyse multipostes de conception très simple, dont les coûts d'installation et de fonctionnement (maintenance, entretien) sont notablement réduits par rapport à ceux des installations existantes actuelles, et dont les caractéristiques de fonctionnement sont optimales en terme de traitement par dialyse.

Un autre objectif de l'invention est de réduire à leur plus simple expression les risques de pollution de l'eau pure, et de faire en sorte qu'un seul patient soit concerné en cas de problème.

Un autre objectif de l'invention est de faciliter et d'écourter les interventions du personnel traitant.

A cet effet, l'invention vise une installation de dialyse multipostes comprenant :
- une pluralité de générateurs de dialyse, chacun destiné à être affecté à un malade,
- des moyens d'alimentation en eau de chaque générateur de dialyse, appelés à être connectés à une arrivée d'eau brute et comprenant une conduite principale d'alimentation, des branches de distribution d'eau vers chacun desdits générateurs de dialyse raccordées à ladite conduite principale, et au moins une pompe disposée sur la conduite principale en amont des branches de distribution
- et des moyens de purification par osmose inverse adaptés pour permettre de délivrer une eau, dite pure, de qualité appropriée à la dialyse, et comprenant, sur chaque branche de distribution, une unité d'osmose inverse disposée sur ladite branche en amont du générateur de dialyse et renfermant au moins une membrane semi-perméable d'osmose inverse.

Selon l'invention, cette installation de dialyse se caractérise en ce que :
- la conduite principale est obturée à son extrémité aval, et la pompe est adaptée pour élever la pression de l'eau circulant dans ladite conduite principale à une pression Pₒₛₘ, supérieure à la pression osmotique de l'eau, apte à permettre un traitement par osmose inverse,
- chaque unité d'osmose inverse de chaque branche de distribution est agencée pour recevoir l'eau à la pression Pₒₛₘ sur une entrée et pour délivrer, d'une part, une eau purifiée sur une première sortie dite sortie d'eau purifiée et, d'autre part, une eau chargée vers une seconde sortie dite sortie de rejet,
- chaque branche de distribution d'eau comprend, en aval de l'unité d'osmose inverse, un connecteur hydraulique et un clapet taré à une pression de tarage prédéterminée Pₐ inférieure à la pression Pₒₛₘ, définissant la pression d'alimentation du générateur de dialyse, ledit connecteur et ledit clapet étant adaptés pour dériver, de la branche de distribution, un débit d'eau d'alimentation à la pression de tarage Pₐ vers le générateur de dialyse.
(Il est à noter que les termes "amont" et "aval" sont utilisés en référence au sens d'écoulement de l'eau).

A l'inverse de la solution constante utilisée systématiquement lors de la réalisation d'installations de dialyse multipostes, et qui consiste à produire en amont, au moyen d'un osmoseur complexe, une eau pure et fragile que l'on s'évertue à conserver dans son état originel en la transportant parfois sur des centaines de mètres, le premier concept de l'invention a donc été de viser à produire l'eau pure directement à proximité de chaque générateur.

Sur la base de ce concept, l'invention a alors consisté à placer au moins une petite membrane d'osmose inverse à proximité immédiate ou à l'intérieur de chaque générateur de dialyse, à alimenter ces membranes au moyen de canalisations distribuant de l'eau brute du réseau simplement filtrée pour retenir les matières en suspension, à une pression Pₒₛₘ apte à permettre un traitement par osmose inverse, et enfin à alimenter tous les générateurs de dialyse à une même pression Pₐ inférieure à la pression Pₒₛₘ.

Par rapport aux installations de dialyse multipostes actuelles, et en tenant compte du fait que les membranes d'osmose inverse constituent un barrage parfait pour produire une eau pure de qualité appropriée à la dialyse, les avantages de l'installation selon l'invention sont multiples et incontestables.

En effet, et en premier lieu, sur le plan de la santé des malades et du travail du personnel soignant et de maintenance, l'installation présente les avantages suivants :
- alimentation de chaque générateur avec une eau pure produite à proximité immédiate dudit générateur et, par conséquent, suppression des risques de développement de germes dans cette eau pure du fait de la faible longueur de canalisation devant être parfaitement désinfectée relativement aux longueurs des boucles actuelles à désinfecter,
- réduction optimale du matériel sujet à usure et donc susceptible de tomber en panne, ce matériel se réduisant, en effet, à la seule pompe,
- absence de bras morts soumis à des risques de contamination et de développements bactériens,
- suppression pour tout le personnel concerné du souci constant d'une éventuelle panne de l'osmoseur ou d'une pollution de la boucle d'alimentation,
- suppression pour le personnel et les patients du stress et des risques consécutifs à un dysfonctionnement de l'installation ; en effet, selon l'invention, en cas de dysfonctionnement d'une unité d'osmose inverse, un seul malade est concerné et non l'ensemble des malades tel qu'actuellement,
- suppression pour le personnel du temps à consacrer pour l'entretien, la maintenance et le dépannage ponctuel de l'osmoseur.

De plus, au point de vue de la désinfection de l'installation, outre la suppression tel que précité de l'obligation de désinfecter les canalisations en amont des générateurs de dialyse, ces opérations de désinfection peuvent être notablement simplifiées.

En effet, cette désinfection peut être effectuée en injectant dans la conduite principale un produit désinfectant-détartrant, de type connu actuellement, diluable dans l'eau et ayant la propriété de traverser intégralement les membranes. (A titre d'exemple, des produits de ce type sont commercialisés sous l'appellation "SOLUDIASTER", "STERIDIAL", "DIALOX" (marques déposées).)

Une telle solution véhiculée au moyen de la pompe conduit à détartrer, en premier lieu, les membranes d'osmose, le tartre étant rejeté au niveau de la sortie de rejet de ces dernières. De plus, son efficacité désinfectante et détartrante restant intacte après le passage dans les membranes, cette solution est en outre apte à désinfecter et détartrer les générateurs en même temps que le reste de l'installation.

Par conséquent, en vue de lancer un cycle de désinfection, il n'est pas requis d'arrêter les générateurs de dialyse en fin de séance, ni de préparer le bidon traditionnel de désinfectant sur l'aspiration de chacun desdits générateurs. La seule opération à effectuer consiste, une fois le dernier patient débranché, à injecter dans la conduite principale le produit désinfectant-détartrant, puis de contrôler 30 à 45 mn plus tard qu'il ne reste plus de traces de ce produit dans l'installation.

Par ailleurs, et de façon essentielle, tous les avantages précités sont obtenus par le biais d'une installation présentant, en terme de traitement par dialyse, des caractéristiques de fonctionnement optimales, non égalées par celles des installations de dialyse multipostes actuellement en service.

En effet, et en premier lieu, tous les générateurs de dialyse de cette installation sont alimentés à une même pression, par exemple de 1 bar, déterminée par le tarage des clapets disposés sur chaque branche de distribution et, de ce fait, d'une part, on obtient une rationalisation de la maintenance des pièces hydrauliques et, d'autre part, on s'exempte de tout risque de dérèglement du débit de dilution des concentrés.

De plus, l'eau délivrée au niveau de chaque membrane d'osmose inverse consiste en une eau élevée à une « pression de travail » importante, sensiblement égale, pour toutes les branches de distribution, à la pression Pₒₛₘ fournie par la pompe diminuée de la pression de tarage des clapets disposés sur chacune desdites branches. De ce fait, l'eau osmosée présente une qualité optimale, et se trouve produite avec un débit, et par conséquent une vitesse de circulation importants, aptes à perturber les développements bactériens.

L'installation selon l'invention comporte, en outre, des avantages du point de vue financier, à savoir principalement :
- diminution sensible du coût de l'investissement par surpression de l'osmoseur général et des contraintes liées à la réalisation et à la désinfection de la boucle d'alimentation,
- suppression du coût de maintenance de l'osmoseur général et de l'entretien de la boucle d'alimentation,
- suppression du coût de transfert éventuel des patients vers d'autres sites,
- ...

En dernier lieu, et tel que précité, le seul matériel sujet à usure mécanique consiste en la pompe, et il peut être facilement remédié à limiter de façon optimale tout risque de panne de ladite pompe en prévoyant de façon avantageuse deux pompes connectées en parallèle, lesdites pompes étant destinées à être alternativement mises en service pendant un laps de temps prédéterminé.

Il convient de souligner qu'il a été envisagé avant la date de mise au point de l'invention, de réaliser une installation de dialyse telle que décrite dans DE 198 264 32 visant à produire l'eau pure à proximité immédiate de générateurs de dialyse, et comportant à cet effet, les moyens définis dans le préambule de la revendication 1 de la présente demande.

Toutefois, l'installation multipostes à boucle principale d'alimentation ouverte telle que décrite dans ce brevet, s'avère inapte à fonctionner même de façon incorrecte, et il n'est décrit, ni suggéré dans ce document de solutions visant à rendre exploitable une telle installation. De ce fait, cette divulgation semble avoir scellé le sort du concept visant à produire de l'eau pure à proximité de générateurs de dialyse, et conforté la profession dans son opinion que seules des évolutions des installations de dialyse actuelles sont susceptibles de permettre de répondre aux exigences de plus en plus draconiennes qu'imposent les traitements par dialyse.

Selon un mode de réalisation avantageux, l'installation de dialyse selon l'invention comprend une cuve-tampon dotée de moyens de régulation du niveau d'eau à l'intérieur de ladite cuve, intercalée sur la conduite principale entre les moyens de filtration et la pompe, et des moyens de recyclage adaptés pour permettre de recycler vers cette cuve-tampon l'eau purifiée de chaque branche d'alimentation d'un générateur de dialyse non dérivée vers ledit générateur.

Une telle disposition présente plusieurs avantages. En effet, et en premier lieu, elle limite de façon notable le volume d'eau nécessaire au fonctionnement de l'installation du fait que l'eau pure non consommée par les générateurs de dialyse est recyclée vers la cuve-tampon et réutilisée mélangée avec l'eau brute provenant de l'arrivée d'eau. De plus, elle permet une vitesse de circulation de l'eau plus élevée, ce qui est toujours souhaitable, et donc l'utilisation de membranes d'osmose standard à grand débit aptes à donner satisfaction concernant la qualité de l'eau pure produite, quelles que soient les conditions de fonctionnement (générateurs de types différents n'ayant pas les mêmes exigences en débit d'eau instantané, différence de besoins des générateurs en terme de débit d'eau de rinçage par rapport au débit d'eau de dialyse, différence de la température de l'eau brute influant directement sur le débit d'eau osmosée, ...).

De plus, ce recyclage conduit à abaisser la concentration en sels minéraux et donc 1a dureté de l'eau délivrée vers les unités d'osmose inverse du fait que cette eau est constituée d'un mélange d'eau brute et d'eau purifiée. De ce fait, et en premier lieu, la qualité de l'eau purifiée produite est améliorée et les risques d'entartrage des membranes d'osmose sont réduits.

Dans la pratique, il a été constaté que lorsque l'eau brute provenant du réseau présentait une dureté faible ou peu élevée, et moyennant des cycles de désinfection et de détartrage fréquents (classiquement après chaque séance de traitement), l'installation de dialyse pouvait être dépourvue de moyens d'adoucissement sans risque d'entartrage des membranes d'osmose inverse.

De ce fait, le recyclage conduit à supprimer le coût d'investissement des moyens d'adoucissement ainsi que les frais de maintenance (dépannage, entretien) de ces derniers.

Toutefois, dans le cas de réseaux d'eau brute présentant une dureté importante, l'installation de dialyse peut avantageusement être équipée de moyens d'adoucissement d'eau intercalés sur la conduite principale et comportant tout type de détartreur connu en soi (chimique, physique, ou magnétique).

Par ailleurs, la cuve-tampon peut avantageusement être utilisée pour les cycles de désinfection et détartrage. En effet, les produits désinfectants actuels (tels que ceux précités) sont des produits liquides présentant la propriété de se diluer instantanément dans l'eau de façon homogène.

De ce fait, en vue d'un cycle de désinfection-détartrage, le liquide peut être directement introduit dans la cuve-tampon, et le seul geste à faire par le personnel soignant pour lancer ce cycle consiste, une fois le dernier patient débranché, à verser un bidon de liquide désinfectant-détartrant dans la cuve-tampon.

Toutefois, en variante, l'installation de dialyse peut également avantageusement comprendre, en vue de la désinfection, un récipient renfermant un produit désinfectant-détartrant, raccordé à la conduite principale, et des moyens d'injection dudit produit dans ladite conduite principale.

Selon un mode de réalisation avantageux, chaque unité d'osmose inverse renferme deux membranes d'osmose inverse connectées en série, la sortie de rejet de la seconde membrane étant raccordée aux moyens de recyclage de façon que l'eau de rejet de ladite seconde membrane soit recyclée vers la cuve-tampon.

Ainsi, le fait de procéder à une bi-osmose inverse conduit à augmenter la qualité de l'eau pure produite et fournit une double sécurité. De plus, cette bi-osmose ne conduit pas à une augmentation du volume d'eau nécessaire au fonctionnement de l'installation du fait que l'eau de rejet de la seconde membrane, qui est une eau purifiée par la première membrane, est recyclée vers la cuve-tampon et donc réutilisée.

Par ailleurs, de façon avantageuse, l'installation de dialyse comprend un filtre bactérien intercalé sur chaque branche d'alimentation d'un générateur de dialyse, en aval du connecteur hydraulique. Un tel filtre bactérien a pour but de se garantir contre un éventuel retour de germes vers les générateurs de dialyse, en provenance des moyens de recyclage, notamment pendant l'arrêt de l'installation.

De façon avantageuse, selon l'invention, chaque unité d'osmose inverse intègre des moyens de mesure et d'affichage de la pression de l'eau purifiée. De tels moyens de mesure et d'affichage permettent au personnel soignant, dans l'éventualité où un signal d'alarme de manque d'eau est généré au niveau d'un générateur de dialyse, de vérifier si la pression d'alimentation en eau pure est correcte et donc de déterminer le matériel à l'origine de la panne (unité d'osmose inverse ou générateur de dialyse).

De plus, de façon avantageuse, selon l'invention, chaque unité d'osmose inverse intègre des moyens de mesure des caractéristiques électriques de l'eau purifiée et des moyens d'indication visuelle et/ou sonore de la valeur de ces caractéristiques électriques.

De tels moyens de mesure permettent d'avertir le personnel soignant si les caractéristiques électriques de l'eau pure (résistivité ou conductivité ou mieux encore taux de réjection) représentatives de la qualité de cette dernière ont des valeurs correspondant à l'exigence de qualité requise. Les valeurs des mesures peuvent, classiquement, être directement affichées au moyen d'un écran à cristaux liquides. L'information peut également être fournie au moyen de deux diodes, par exemple verte et rouge, représentatives respectivement l'une d'une qualité d'eau normale et l'autre d'une qualité d'eau hors norme. Ces moyens visuels peuvent, en outre, être doublés par une alarme sonore.

Selon un mode de réalisation avantageux, chaque connecteur hydraulique est porté par un générateur de dialyse et comprend un corps rigide délimitant un canal interne agencé pour assurer la circulation continue de l'eau dans la branche de distribution et une prise de dérivation débouchant dans ledit canal interne et agencée pour communiquer avec l'entrée d'eau du générateur de dialyse.

Par ailleurs, de façon avantageuse, selon l'invention, chaque unité d'osmose inverse intègre le clapet taré, la branche de distribution d'eau vers chaque générateur de dialyse comportant, au niveau de la liaison entre lesdits générateur et unité, deux minces tuyaux connectés d'une part au connecteur hydraulique et d'autre part à des raccords disposés sur l'unité. Une telle unité d'osmose inverse peut ainsi consister en un simple coffre accolé au générateur de dialyse ou fixé au mur derrière celui-ci, ou même intégré dans ledit générateur, la liaison entre lesdits générateur et coffret étant réalisée au moyen de deux simples tuyaux souples de faible diamètre à connexion rapide.

Selon un mode de réalisation avantageux, chaque pompe est adaptée pour élever la pression de l'eau filtrée à une pression Pₒₛₘ de 10 à 12 bars, chaque clapet des branches de distribution d'eau vers les générateurs de dialyse étant taré à une pression de l'ordre de 0,6 à 1 bar.

De plus, de façon avantageuse, selon l'invention, l'installation comprend des moyens de filtration constitués d'un filtre apte à retenir les matières en suspension d'une taille supérieure à 5 microns, d'un filtre à charbon actif, et d'un filtre apte à retenir les matières en suspension d'une taille supérieure à 1 micron.

D'autres caractéristiques, buts et avantages de l'invention, ressortiront de la description détaillée qui suit en référence aux dessins annexés qui en représentent à titre d'exemples non limitatifs deux modes de réalisation préférentiels. Sur ces dessins :
- la figure 1 est une vue d'une installation de dialyse conforme à l'invention représentée en milieu hospitalier,
- la figure 2 est une vue schématique de cette installation de dialyse,
- et la figure 3 est une vue schématique d'une variante de réalisation d'une installation de dialyse selon l'invention.

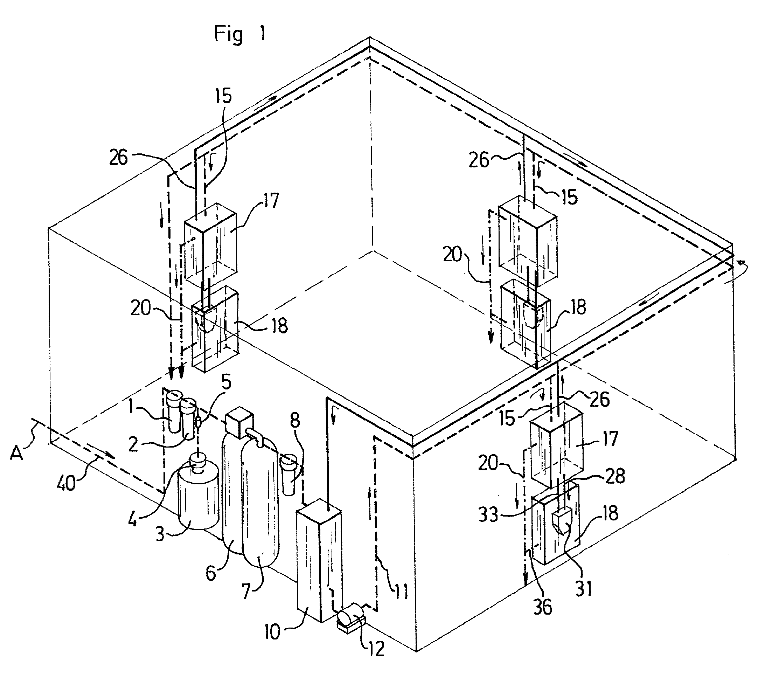

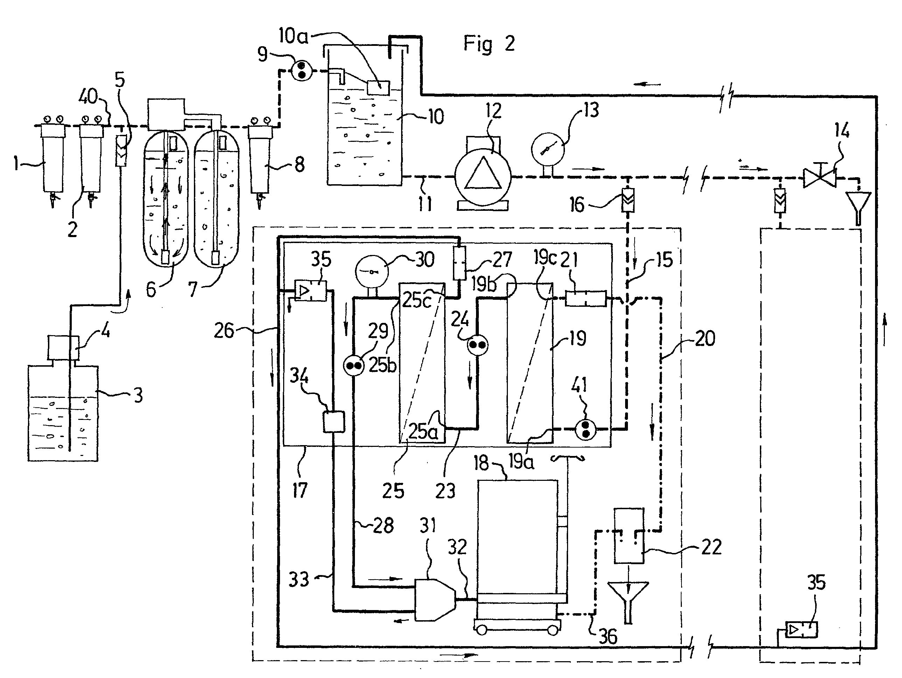

L'installation de dialyse représentée à titre d'exemple aux figures 1 et 2 comprend en premier lieu, intercalés sur une conduite 40 connectée à une arrivée d'eau brute A, des moyens de filtration et d'adoucissement de ladite eau brute, consistant successivement en :
- un filtre 1 pour retenir les impuretés en suspension d'une taille supérieure à 5 microns,
- un deuxième filtre 2 à charbon actif,
- deux adoucisseurs duplex 6, 7 pour éliminer certains sels (en particulier sels de calcium et de magnésium) qui peuvent être remplacés par un détartreur magnétique,
- et un filtre 8 pour retenir les impuretés en suspension d'une taille supérieure à 1 micron.

Cette installation comprend, en outre, intercalé sur la conduite 40, entre le filtre à charbon actif 2 et les adoucisseurs 6, 7, un récipient 3 renfermant un produit désinfectant-détartrant apte à être injecté dans ladite conduite, au moyen d'une pompe doseuse 4, par l'intermédiaire d'une canalisation connectée à la conduite 40 au moyen d'un raccord rapide.

Cette installation comprend également une cuve-tampon 10 dans laquelle débouche l'extrémité aval de la conduite 40, ladite cuve étant équipée d'une vanne à flotteur 10a de régulation du niveau (ou d'une électro-vanne commandée par des niveaux haut et bas), et faisant office de disconnecteur hydraulique vis-à-vis du réseau public.

En outre, une sonde 9 apte à mesurer les caractéristiques électriques (conductivité ou résistivité) de l'eau brute filtrée est intercalée sur la conduite 40, en amont de la cuve-tampon 10.

Cette installation comporte ensuite une conduite principale 11 sur laquelle est intercalée une pompe haute pression 12 adaptée pour distribuer l'eau à une pression constante (10 à 12 bars), contrôlable au moyen d'un manomètre 13, grâce à un système classique de by-pass de retour d'eau vers la cuve-tampon (non représenté).

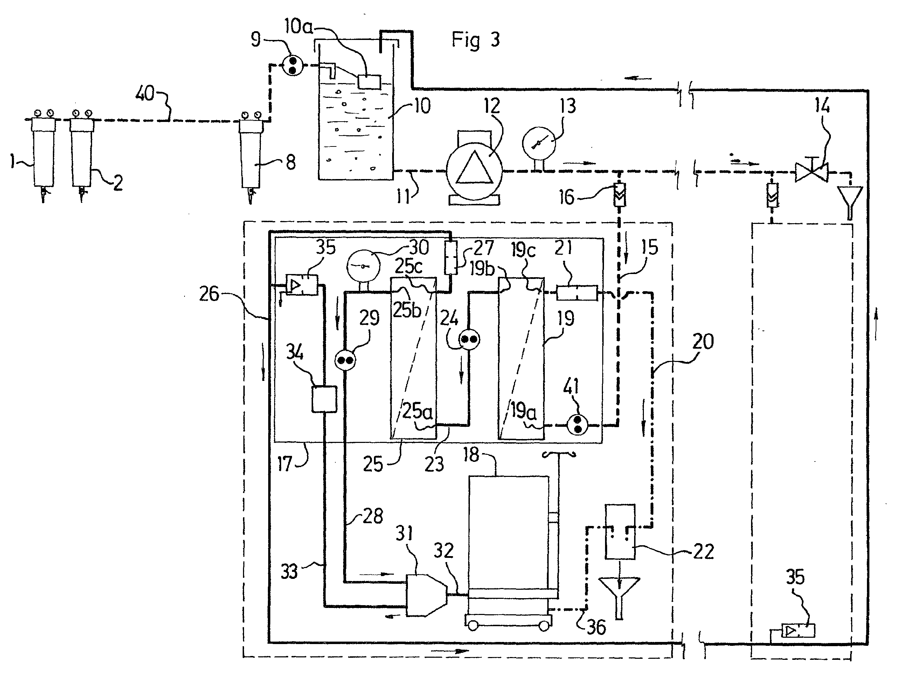

Cette conduite principale 11 est adaptée pour alimenter tous les postes de dialyse et s'étend à cet effet, tel que représenté à la figure 1, sur le pourtour de la salle de traitement, et comporte une vanne de purge ou de rinçage 14 à son extrémité aval (il est à noter que sur les figures seulement 3 postes de dialyse (figure 1) ou 2 postes de dialyse (figures 2 et 3) ont été représentés pour des questions de clarté des dessins. Il est par contre évident que les salles de traitement comportent généralement un nombre de postes de dialyse beaucoup plus élevé).

Au niveau de chaque poste de dialyse, l'installation comporte une branche 15 de distribution d'eau vers un coffret d'osmose inverse 17 d'alimentation d'un générateur de dialyse 18, ladite branche étant connectée à la conduite principale 11 par l'intermédiaire d'un raccord rapide 16 ou d'une vanne.

Chacun des coffrets d'osmose inverse 17 intègre, en premier lieu, deux petites membranes d'osmose inverse 19, 25 disposées en série, la sortie d'eau purifiée 19b de la première membrane 19 étant reliée à l'entrée d'eau 25a de la seconde membrane 25 par une canalisation 23.

La sortie d'eau de rejet 19c de la première membrane 19 est quant à elle, de façon classique, reliée à l'égout par une canalisation 20 sur laquelle est intercalé un limiteur de débit 21, et débouchant dans un boîtier de disconnexion hydraulique 22.

La sortie d'eau purifiée 25b de la seconde membrane 25 est quant à elle reliée par un tuyau souple 28 à un connecteur hydraulique 31 porté par le générateur de dialyse 18. Ce connecteur hydraulique 31 est du type décrit dans le brevet français FR 2.704.150, auquel on se réfèrera pour plus de détails, et comporte donc, en premier lieu, un corps rigide doté d'un canal interne agencé pour assurer la circulation continue de l'eau purifiée provenant du tuyau souple 28 vers un second tuyau souple 33 raccordé au coffret d'osmose inverse 17. Ce connecteur hydraulique 31 comprend, en outre, tel que décrit dans le brevet français précité, une prise de dérivation 32 débouchant dans le canal interne et agencée pour communiquer avec l'entrée d'eau du générateur de dialyse 18.

Dans le prolongement du second tuyau souple 33, le coffret d'osmose inverse 17 intègre, en outre, un filtre bactérien 34, et un clapet 35 taré à une pression Pₐ de 1 bar déterminant la pression d'alimentation du générateur de dialyse 18.

De plus, l'eau non utilisée par le générateur de dialyse 18 et ayant circulé dans le canal interne du connecteur hydraulique 31, ainsi que l'eau de rejet délivrée au niveau de la sortie de rejet 25c de la seconde membrane 25 et s'écoulant au travers d'un limiteur de débit 27, sont délivrées vers une canalisation de recyclage 26, adaptée pour collecter et recycler vers la cuve-tampon 10 les eaux purifiées similaires de tous les postes de dialyse.

Chaque coffret d'osmose inverse 17 intègre, par ailleurs, un manomètre 30 disposé en aval de la sortie d'eau purifiée 25b de la seconde membrane 25 et adapté pour permettre de contrôler la pression de l'eau purifiée en sortie de ladite seconde membrane.

Chacun de ces coffrets d'osmose inverse 17 comporte enfin trois sondes 41, 24, 29 disposées respectivement en amont de l'entrée d'eau 19a de la première membrane 19 et en aval des sorties d'eau purifiées 19b, 25b des première et seconde membranes 19 et 25, et destinées à permettre de mesurer les caractéristiques électriques (conductivité ou résistivité et taux de réjection) de l'eau en sortie desdites membranes et d'en informer le personnel soignant par tout moyen d'indication tel que diodes, écrans à cristaux liquides, bips sonores.

En dernier lieu, et de façon classique, la sortie d'eau de chaque générateur de dialyse 18 est raccordée au disconnecteur hydraulique 22 par une canalisation 36.

L'installation de dialyse représentée à la figure 3 est similaire à celle décrite ci-dessus, la seule différence résultant des possibilités qu'offre la conception de l'installation selon l'invention, à savoir la suppression du récipient 3 et de la pompe doseuse 4, ainsi que des adoucisseurs 6, 7. Selon cette version, le produit désinfectant-détartrant est introduit directement dans la cuve-tampon 10, tandis que l'absence d'adoucisseurs 6, 7, à la condition que la dureté de l'eau brute ne soit pas trop élevée, est palliée par le recyclage d'une partie de l'eau purifiée vers la cuve-tampon 10 qui conduit à abaisser la dureté de l'eau délivrée vers les membranes 19, 25.

## Revendications

1. Installation de dialyse multipostes comprenant :
- une pluralité de générateurs de dialyse (18), chacun destiné à être affecté à un malade,
- des moyens d'alimentation en eau de chaque générateur de dialyse (18), appelés à être connectés à une arrivée d'eau brute (A) et comprenant une conduite principale d'alimentation (40, 11), des branches de distribution d'eau (15, 28, 33) vers chacun desdits générateurs de dialyse raccordées à ladite conduite principale, et au moins une pompe (12) disposée sur la conduite principale (40, 11) en amont des branches de distribution (15, 28, 33),
- et des moyens de purification par osmose inverse (17) adaptés pour permettre de délivrer une eau, dite pure, de qualité appropriée à la dialyse, et comprenant, sur chaque branche de distribution (15, 28, 33), une unité d'osmose inverse (17) disposée sur ladite branche en amont du générateur de dialyse (18) et renfermant au moins une membrane semi-perméable d'osmose inverse (19, 25), ladite installation de dialyse étant **caractérisée en ce que** :
- la conduite principale (40, 11) est obturée à son extrémité aval, et la pompe (12) est adaptée pour élever la pression de l'eau circulant dans ladite conduite principale à une pression Pₒₛₘ, supérieure à la pression osmotique de l'eau, apte à permettre un traitement par osmose inverse,
- chaque unité d'osmose inverse (17) de chaque branche de distribution (15, 28, 33) est agencée pour recevoir l'eau à la pression Pₒₛₘ sur une entrée (19a, 25a) et pour délivrer, d'une part, une eau purifiée sur une première sortie (19b, 25b) dite sortie d'eau purifiée et, d'autre part, une eau chargée vers une seconde sortie (19c, 25c) dite sortie de rejet,
- chaque branche de distribution d'eau (15, 28, 33) comprend, en aval de l'unité d'osmose inverse (17), un connecteur hydraulique (31) et un clapet (35) taré à une pression de tarage prédéterminée Pₐ inférieure à la pression Pₒₛₘ, définissant la pression d'alimentation du générateur de dialyse (18), ledit connecteur et ledit clapet étant adaptés pour dériver, de la branche de distribution (15, 28, 33), un débit d'eau d'alimentation à la pression de tarage Pₐ vers le générateur de dialyse (18).

2. Installation de dialyse selon la revendication 1, **caractérisée en ce qu'**elle comprend une cuve-tampon (10) dotée de moyens (10a) de régulation du niveau d'eau à l'intérieur de ladite cuve, intercalée sur la conduite principale (40, 11) en amont de la pompe (12), et des moyens de recyclage (26) adaptés pour permettre de recycler vers cette cuve-tampon (10) l'eau purifiée de chaque branche de distribution d'eau (15, 28, 33) d'un générateur de dialyse (18) non dérivée vers ledit générateur.

3. Installation de dialyse selon la revendication 2, **caractérisée en ce que** chaque unité d'osmose inverse (17) renferme deux membranes d'osmose inverse (19, 25) connectées en série, la sortie de rejet (25c) de la seconde membrane (25) étant raccordée aux moyens de recyclage (26) de façon que l'eau de rejet de ladite seconde membrane soit recyclée vers la cuve-tampon (10).

4. Installation de dialyse selon l'une des revendications 2 ou 3, **caractérisée en ce qu'**elle comprend un filtre bactérien (34) intercalé sur chaque branche de distribution d'eau (15, 28, 33) vers un générateur de dialyse (18), en aval du connecteur hydraulique (31).

5. Installation de dialyse selon l'une des revendications précédentes, **caractérisée en ce qu'**elle comprend un récipient (3) renfermant un produit désinfectant-détartrant, raccordé à la conduite principale (40, 11), et des moyens d'injection (4) dudit produit dans ladite conduite principale.

6. Installation de dialyse selon l'une des revendications précédentes, **caractérisée en ce qu'**elle comprend des moyens d'adoucissement d'eau (6, 7) intercalés sur la conduite principale (40, 11).

7. Installation de dialyse selon l'une des revendications précédentes, **caractérisée en ce que** chaque unité d'osmose inverse (17) intègre des moyens (30) de mesure et d'affichage de la pression de l'eau purifiée.

8. Installation de dialyse selon l'une des revendications précédentes, **caractérisée en ce que** chaque unité d'osmose inverse (17) intègre des moyens de mesure (24, 29, 41) des caractéristiques électriques de l'eau purifiée, et des moyens d'indication visuelle et/ou sonore de la valeur de ces caractéristiques électriques.

9. Installation de dialyse selon l'une des revendications précédentes, **caractérisée en ce que** chaque connecteur hydraulique (31) est porté par un générateur de dialyse (18) et comprend un corps rigide délimitant un canal interne agencé pour assurer la circulation continue de l'eau dans la branche de distribution (15, 28, 33), et une prise de dérivation (32) débouchant dans ledit canal interne et agencée pour communiquer avec l'entrée d'eau du générateur de dialyse.

10. Installation de dialyse selon la revendication 9, **caractérisée en ce que** chaque unité d'osmose inverse (17) intègre le clapet taré (35), la branche de distribution d'eau (15, 28, 33) vers chaque générateur de dialyse (18), comportant, au niveau de la liaison entre lesdits générateur et unité d'osmose inverse, deux minces tuyaux (28, 33) connectés d'une part au connecteur hydraulique (31) et d'autre part à des raccords disposés sur l'unité d'osmose inverse (17).

11. Installation de dialyse selon l'une des revendications précédentes, **caractérisée en ce que** chaque pompe (12) est adaptée pour élever la pression de l'eau à une pression Pₒₛₘ de 10 à 12 bars, chaque clapet (35) des branches de distribution (15, 28, 33) vers les générateurs de dialyse (18) étant taré à une pression Pₐ de l'ordre de 0,6 à 1 bar.

12. Installation de dialyse selon l'une des revendications précédentes, **caractérisée en ce qu'**elle comprend deux pompes (12) connectées en parallèle, lesdites pompes étant destinées à être alternativement mises en service pendant un laps de temps prédéterminé.

13. Installation de dialyse selon l'une des revendications précédentes, **caractérisée en ce qu'**elle comprend des moyens de filtration constitués d'un filtre (1) apte à retenir les matières en suspension d'une taille supérieure à 5 microns, d'un filtre (2) à charbon actif, et d'un filtre (8) apte à retenir les matières en suspension d'une taille supérieure à 1 micron.
